# EUROPÄISCHE PATENTSCHRIFT

(11) **EP 3 502 082 B1**
(45) Veröffentlichungstag und Bekanntmachung des Hinweises auf die Patenterteilung: **12.02.2020**
(21) Anmeldenummer: 17209307.2
(22) Anmeldetag: 21.12.2017
(51) Int. Cl.: C07C 51/14, C07C 53/126, B01J 31/22, B01J 31/24, B01J 31/30, C07C 53/128

(54) **VERFAHREN ZUR PD-KATALYSIERTEN HYDROXYCARBONYLIERUNG VON DIISOBUTEN: EINFLUSS DES LÖSUNGSMITTELS**
METHOD FOR THE PD CATALYTIC HYDROXYCARBONYLATION OF DI-ISOBUTENE: INFLUENCE OF THE SOLVENT
PROCÉDÉ D'HYDROXYCARBONYLATION DE DIISOBUTÈNE CATALYSÉE PAR LE PALLADIUM : INFLUENCE DU SOLVANT

(43) Veröffentlichungstag der Anmeldung: 26.06.2019
(73) Patentinhaber: Evonik Operations GmbH, 45128 Essen (DE)
(72) Erfinder: SANG, Rui, 252000 Liaocheng City, Shandong Province (CN); KUCMIERCZYK, Peter, 44652 Herne (DE); DONG, Kaiwu, 236800 Bo Zhou (CN); JACKSTELL, Ralf, 10106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- WO-A1-2014/005854
- DE-C- 887 644
- GB-A- 1 595 037
- US-A- 3 641 074
- US-A- 3 968 133

## Beschreibung

Die Erfindung betrifft ein Verfahren zur Pd-katalysierten Hydroxycarbonylierung von Diisobuten: Einfluss des Lösungsmittels.

Carbonsäuren einschließlich Propionsäure, Adipinsäure und Fettsäuren werden bei der Herstellung von Polymeren, Pharmazeutika, Lösungsmitteln und Lebensmittelzusatzstoffen verwendet. Zu den Routen, welche zu Carbonsäuren führen, gehören im Allgemeinen die Oxidation von Kohlenwasserstoffen, Alkoholen oder Aldehyden, die oxidative Spaltung von Olefinen durch Ozonolyse, die Hydrolyse von Triglyceriden, Nitrilen, Estern oder Amiden, die Carboxylierung von Grignard- oder Organolithium-Reagenzien und die Halogenierung und anschließende Hydrolyse von Methylketonen in der Haloform-Reaktion.

Die Hydrocarboxylierung von Olefinen ist eine sehr aussichtsreiche und umweltfreundliche Methode zum Erhalt von Carbonsäuren. Die Herstellung von Essigsäure erfolgt durch Carbonylierung von Methanol, die mit lodid durchgeführt wird. Bei der Koch-Reaktion wird die Addition von Wasser und Kohlenmonoxid an Alkene durch starke Basen katalysiert. Diese Methode funktioniert gut bei Alkenen, die sekundäre und tertiäre Carbokationen bilden, z.B. Isobutylen zu Pivalinsäure. Die mit gleichzeitiger Addition von CO und H₂O an Alkene/Alkine verlaufende Hydrocarboxylierung stellt eine direkte und bequeme Methode zur Synthese von Carbonsäuren zur Verfügung.

WO2014/ 005854 offenbart ein zweistufiges Verfahren zur Herstellung von 3,5,5-Trimethylhexansäure aus Diisobutylen. Der Erfindung lag die Aufgabe zugrunde, ein Verfahren bereitzustellen, welches bei der Pd-katalysierten Hydroxycarbonylierung von Diisobuten (DIBN) einen guten Umsatz liefert. Die Umsetzung soll hierbei in einem Schritt erfolgen.

Gelöst wird die Aufgabe durch ein Verfahren nach Anspruch 1.

Verfahren umfassend die Verfahrensschritte:
a) Zugabe von Diisobuten,
b) Zugabe einer Verbindung, welche Pd umfasst, wobei das Pd in der Lage ist einen Komplex auszubilden,
c) Zugabe des Liganden L1: 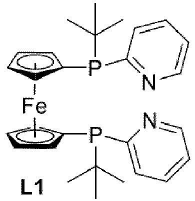
d) Zugab von Essigsäure,
e) Zuführen von CO,
f) Erhitzen des Reaktionsgemisches, so dass das Diisobuten zur Verbindung **P1** umgesetzt wird:

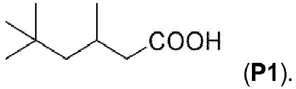

In einer Variante des Verfahrens ist die Verbindung im Verfahrensschritt b) ausgewählt aus: PdCl₂, PdBr₂, Pd(acac)₂, Pd(dba)₂ (dba = Dibenzylidenaceton), PdCl₂(CH₃CN)₂.

In einer Variante des Verfahrens ist die Verbindung im Verfahrensschritt b) Pd(acac)₂.

In einer Variante des Verfahrens umfasst das Verfahren den zusätzlichen Reaktionsschritt g):
g) Zugabe von p-Toluolsulfonsäure (PTSA).

In einer Variante des Verfahrens wird das Reaktionsgemisch im Verfahrensschritt f) auf eine Temperatur im Bereich von 80 °C bis 160 °C erhitzt.
Vorzugsweise auf eine Temperatur im Bereich von 100 °C bis 140 °C.

In einer Variante des Verfahrens erfolgt das Zuführen von CO im Verfahrensschritt e) so, dass die Reaktion bei einem CO-Druck im Bereich von 20 bar bis 60 bar verläuft. Vorzugsweise im Bereich von 30 bar bis 50 bar.

Im Folgenden wird die Erfindung anhand eines Ausführungsbeispiels näher erläutert.

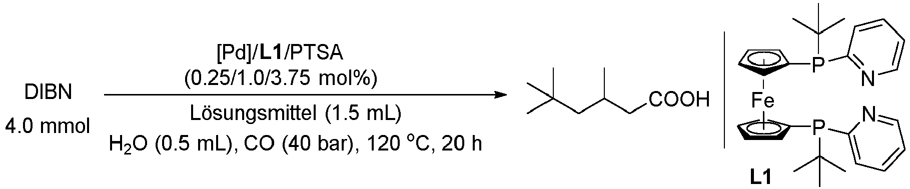

Ein 4-mL-Vial wurde mit [Pd(acac)₂] (3,05 mg, 0,25 mol-%), L1 (20,64 mg, 1,0 mol-%), PTSA*H₂O (28,5 mg, 3,75 mol-%) und einem im Ofen getrockneten Rührstab beschickt. Dann wird das Vial mit Septen (PTFE-beschichteter Styrol-Butadien-Kautschuk) und Phenolharzdeckel verschlossen. Das Vial wird dreimal evakuiert und mit Argon wieder befüllt. Mit einer Spritze wurden H₂O (0,5 ml), Essigsäure (1,5 ml) und Diisobuten (DIBN) (4,0 mmol) in das Vial gegeben. Das Vial wurde in eine Legierungsplatte gestellt, die in einen Autoklaven (300 ml) der Reihe 4560 von Parr Instruments unter Argonatmosphäre überführt wurde. Nach dreimaligem Spülen des Autoklaven mit CO wurde der CO-Druck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Dann wurde Isooctan (100 ul) als interner Standard zugegeben. Der Umsatz wurde durch GC-Analyse gemessen.

Der oben beschriebene Versuch wurde unter Variation des Lösungsmittels wiederholt. Alle anderen Parameter wurden beibehalten.

Die Ergebnisse sind in der nachfolgenden Tabelle zusammengestellt.

| Eintrag | Lösungsmittel | Umsatz (%) |
|---|---|---|
| 1 | 3,5,5-Trimethylhexansäure | 88 |
| 2 | Ameisensäure | 48 |
| 3* | Essigsäure | >99 |

| | | |
|---|---|---|
| * erfindungsgemäßes Verfahren | | |

Wie die Versuchsergebnisse zeigen wird die Aufgabe durch das erfindungsgemäße Verfahren gelöst.

## Patentansprüche

1. Verfahren umfassend die Verfahrensschritte:
a) Zugabe von Diisobuten,
b) Zugabe einer Verbindung, welche Pd umfasst, wobei das Pd in der Lage ist einen Komplex auszubilden,
c) Zugabe des Liganden **L1:** 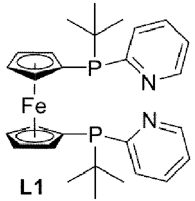
d) Zugab von Essigsäure,
e) Zuführen von CO,
f) Erhitzen des Reaktionsgemisches, so dass das Diisobuten zur Verbindung **P1** umgesetzt wird:

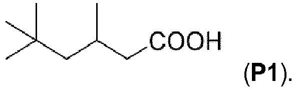

2. Verfahren nach Anspruch 1,
wobei die Verbindung im Verfahrensschritt b) ausgewählt ist aus:
PdCl₂, PdBr₂, Pd(acac)₂, Pd(dba)₂ (dba = Dibenzylidenaceton), PdCl₂(CH₃CN)₂.

3. Verfahren nach einem der Ansprüche 1 oder 2,
wobei das Verfahren den zusätzlichen Reaktionsschritt g) umfasst:
g) Zugabe von p-Toluolsulfonsäure.

4. Verfahren nach einem der Verfahrensschritte 1 bis 3,
wobei das Reaktionsgemisch im Verfahrensschritt f) auf eine Temperatur im Bereich von 80 °C bis 160 °C erhitzt wird.

5. Verfahren nach einem der Verfahrensschritte 1 bis 4,
wobei das Zuführen von CO im Verfahrensschritt e) so erfolgt, dass die Reaktion bei einem CO-Druck im Bereich von 20 bar bis 60 bar verläuft.

## Claims

1. Process comprising the process steps of:
a) addition of diisobutene,
b) addition of a compound comprising Pd, wherein the Pd is capable of forming a complex,
c) addition of the ligand **L1**: 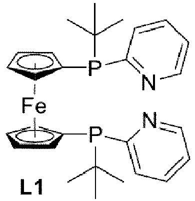
d) addition of acetic acid,
e) feeding in CO,
f) heating the reaction mixture such that the diisobutene is converted to the compound **P1:**

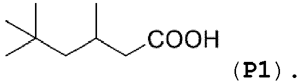

2. Process according to Claim 1,
wherein the compound in process step b) is selected from:
PdCl₂, PdBr₂, Pd(acac)₂, Pd(dba)₂ (dba = dibenzylideneacetone), PdCl₂(CH₃CN)₂.

3. Process according to either of Claims 1 and 2,
wherein the process comprises the additional reaction step g):
g) addition of p-toluenesulfonic acid.

4. Process according to any of process steps 1 to 3, wherein the reaction mixture is heated to a temperature in the range from 80°C to 160°C in process step f).

5. Process according to any of process steps 1 to 4, wherein the CO is fed in in process step e) such that the reaction proceeds under a CO pressure in the range from 20 bar to 60 bar.

## Revendications

1. Procédé comprenant les étapes de procédé :
a) ajout de diisobutène,
b) ajout d'un composé, qui comprend du Pd, le Pd étant apte à former un complexe,
c) ajout du ligand L1 : 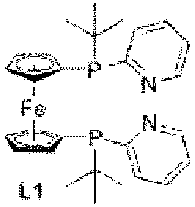
d) ajout d'acide acétique,
e) introduction de CO,
f) chauffage du mélange réactionnel, de sorte que le diisobutène est transformé en composé P1 :

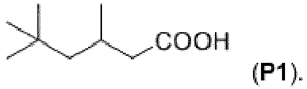

2. Procédé selon la revendication 1,
le composé dans l'étape de procédé b) étant choisi parmi :
PdCl₂, PdBr₂, Pd(acac)₂, Pd(dba)₂ (dba = dibenzylydèneacétone), PdCl₂(CH₃CN)₂.

3. Procédé selon l'une quelconque des revendications 1 et 2,
le procédé comprenant l'étape de réaction supplémentaire g) :
g) ajout d'acide p-toluènesulfonique.

4. Procédé selon l'une quelconque des étapes de procédé 1 à 3,
le mélange réactionnel dans l'étape de procédé f) étant chauffé à une température dans la plage de 80 °C à 160 °C.

5. Procédé selon l'une quelconque des étapes de procédé 1 à 4,
l'introduction de CO dans l'étape de procédé e) étant réalisée de sorte que la réaction a lieu à une pression de CO dans la plage de 20 bars à 60 bars.
